# EUROPEAN PATENT APPLICATION

(11) **EP 4 417 172 A1**
(43) Date of publication of application: **21.08.2024**
(21) Application number: 22895256.0
(22) Date of filing: 03.10.2022
(51) Int. Cl.: A61F 13/15, A61F 13/49, A61F 13/53, A61F 13/539, A61F 13/56

(54) **WEARING ARTICLE AND METHOD OF MANUFACTURING SAME**

(30) Priority: 16.11.2021 JP 2021186702
(71) Applicant: Zuiko Corporation, Ibaraki-shi Osaka 5670082 (JP)
(72) Inventor: UMEBAYASHI, Toyoshi, Osaka 5670082 (JP); YAMAMOTO, Toshinori, Osaka 5300047 (JP)
(74) Representative: Hasegawa, Kan
(86) International application number: PCT/JP2022/036910
(87) International publication number: WO 2023/089970

(57) **Abstract**

Provided are a wearing article and a method for manufacturing the same with which it is possible to form a concave shape in a portion positioned on the ventral side without forming a convex shape or a concave shape on the peripheral edge of the portion positioned on the dorsal side, and improve wearing comfort and appearance.

The present invention is provided with: (a) a girth member (12) having side edges (12a, 12b); (b) an absorbent body (14) in which a cutout part (14p) is formed in the intermediate portion of one end edge thereof (14a), the absorbent body (14) being joined to the girth member (12) so that the other end edge (14b) overlaps with the girth member (12) and the intermediate portion between the end edges (14a, 14b) overlaps with one of the side edges (12a) of the girth member (12); and (c) fastening members (16a, 16b, 16c) enabling the end parts (12m, 12n) of the girth member (12) to repeatedly fasten to and separate from a portion of the absorbent body (14) adjacent to the one end edge (14a). A portion (12c) of the girth member (12) adjacent to the other side edge (12b) is folded back so as to cover the other end edge (14b) of the absorbent body (14).

## Description

### TECHNICAL FIELD

The present invention relates to a wearing article and a method of manufacturing the same, and more particularly to a wearing article with a concave peripheral edge on the front side and a method of manufacturing the same.

### BACKGROUND ART

A wearing article for a newborn baby with a concave peripheral edge on the front side has been proposed so as not to touch the navel of the newborn baby whose umbilical cord has just been cut.

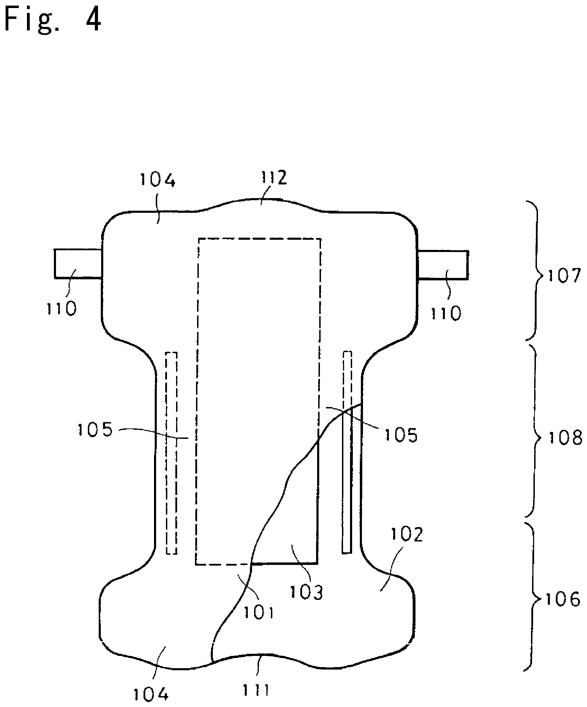

For example, in a wearing article shown in a developed view of FIG. 4, an absorbent body 103 is disposed between a water-permeable top sheet 101 and a water-impermeable back sheet 102 so as to cross the crotch region 108. Flap portions 104 and 105 are formed by front and back sheets 101 and 102 extending outward from the outer peripheral edge of absorbent body 103. A fastening piece 110 having a pressure-sensitive adhesive surface is attached to the flap portion 104 of the dorsal side region 107. A recessed part 111 is formed in a front region 106 located on the front side of the wearer, and a protrusion 112 is formed in a dorsal side region 107 located on the rear side of the wearer (see, for example, Patent Literature 1).

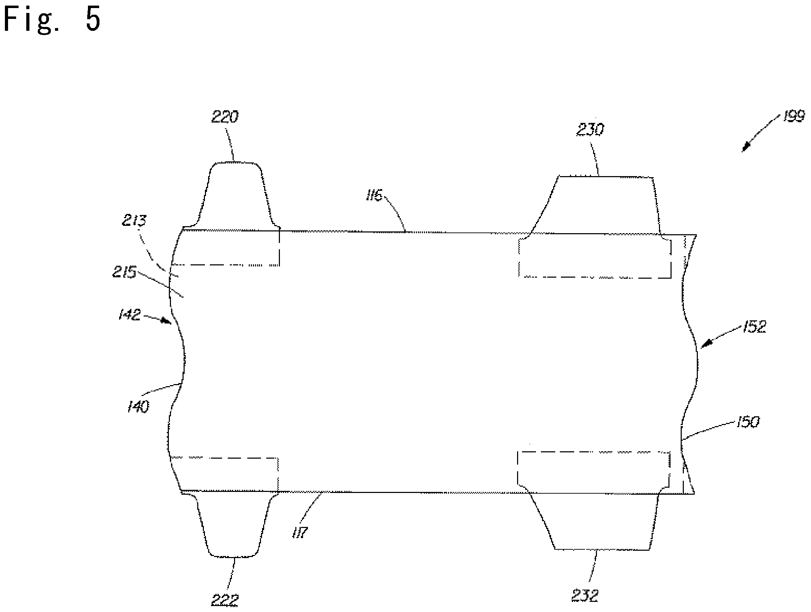

FIG. 5 is a plan view showing another wearing article 199 in a developed state. As shown in FIG. 5, this wearing article 199 has ears 220, 222, 230, 232 joined between a back sheet 213 and a top sheet 215 so as to protrude from longitudinal edge parts 116, 117. The front terminal edge part 140 of the front section, which is located on the front side of the wearer, has a U-shaped cutout part 142. The rear terminal edge part 150 disposed on the rear side of the wearer has a U-shaped protruding cut portion 152 (see, for example, Patent Literature 2).

### CITATION LIST

### PATENT LITERATURE

[Patent Literature 1] JP 62-136505 U1
[Patent Literature 2] JP 4932738 B2

### SUMMARY OF INVENTION

### TECHNICAL PROBLEM

These wearing articles are made by cutting a continuous body in which multiple regions where the wearing article is planned to be formed are connected in a direction connecting the front part placed on the ventral side of the wearer and the rear part placed on the dorsal side of the wearer, and by doing so, corresponding contour shapes are formed in the front part and the rear part. Therefore, if a concave peripheral edge is formed in the front part, the peripheral edge of the rear part is formed in a convex shape, and the convex part may come into contact with the back, giving a sense of discomfort. Furthermore, if the peripheral edge of the rear portion of the wearing article is formed in a convex or concave shape, the external shape becomes complicated, making it difficult to package the wearing article compactly.

In view of these circumstances, the problem to be solved by the present invention is to provide a wearing article with which it is possible to form a concave shape in a portion positioned on the ventral side without forming a convex shape or a concave shape on the peripheral edge of the portion positioned on the dorsal side, and improve wearing comfort and appearance, and a method of manufacturing the same.

### MEANS FOR SOLVING THE PROBLEM

In order to solve the above problems, the present invention provides a wearing article configured as follows.

The wearing article is provided with (a) a girth member having a pair of side edges extending in a first direction, (b) an absorbent body having a pair of end edges facing each other, and in which a cutout part is formed in the intermediate portion of one end edge thereof, the absorbent body being joined to the girth member so that the other end edge overlaps with the girth member and the intermediate portion between the one end edge and the other end edge overlaps with one of the side edges of the girth member, and (c) fastening members enabling both end parts in the first direction of the girth member to repeatedly fasten to and separate from a portion of the absorbent body adjacent to the one end edge, wherein a portion of the girth member adjacent to the other side edge is folded back so as to cover the other end edge of the absorbent body.

According to the above configuration, a concave shape can be formed in the portion disposed on the ventral side by the cutout part on one end edge of the absorbent body. Since the girth member is folded back, no convex or concave shape is formed on the circumference of the dorsal side of the wearing article. Since the other end edge of the absorbent body and its vicinity are covered by the folded portion of the girth member, it is possible to improve the wearing comfort, keep the exterior simple, keep the beauty, and improve the appearance.

Preferably, another cutout part is formed in an intermediate portion of the other end edge of the absorbent body. A portion of the girth member adjacent to the other side edge is folded back so as to cover the other cutout part.

In this case, by forming another cutout part on the other end edge of the absorbent body, the cutout part on one end edge of the absorbent body that is exposed to the outside can be formed with high accuracy, and a cutout part having a desired shape can be formed efficiently. Even if another cutout part is formed on the other end edge of the absorbent body, the other end edge of the absorbent body and its vicinity are covered by the folded portion of the girth member, so that the other cutout part can be made invisible. Therefore, it is possible to keep the wearing comfort intact, keep the exterior simple, maintain beauty, and improve the appearance.

Moreover, in order to solve the above-mentioned problem, the present invention provides a method of manufacturing a wearing article configured as follows.

The method of manufacturing a wearing article comprises
(i) a girth member continuous body preparation process of preparing a girth member continuous body having a pair of side edges extending in a first direction, and in which a plurality of first regions in which girth members are to be formed are connected in the first direction,
(ii) an absorbent body continuous body preparation process of preparing an absorbent body continuous body in which a plurality of second regions in which absorbent bodies are to be formed are connected in a second direction,
(iii) an absorbent body cutting process of cutting the absorbent body continuous body to form the absorbent body so that a pair of end edges are formed on the absorbent body and a cutout part is formed on the intermediate portion of the one end edge,
(iv) an absorbent body joining process of joining a plurality of the absorbent bodies to the girth member continuous body at intervals from each other in the first direction so that the other end edge of the absorbent body overlaps with the girth member continuous body and the intermediate portion between the one end edge and the other end edge of the absorbent body overlaps with the one side edge of the girth member continuous body,
(v) a folding back process of folding-back a portion of the girth member continuous body adjacent to the other side edge to cover the other end edge of the absorbent body joined to the girth member continuous body,
(vi) a girth member cutting process of cutting the girth member continuously body in which a portion adjacent to the other side edge is folded, to form the girth member having end parts on both sides of the first direction, and
(vii) a fastening member fixing process of fixing a fastening member enabling the end part of the girth member to repeatedly fasten to and separate from a portion of the absorbent body adjacent to the one end edge, to the girth member continuous body or the girth member.

According to the above method, a concave shape can be formed in the portion disposed on the ventral side by the cutout part on one end edge of the absorbent body. Since the girth member is folded back and no convex or concave shape is formed on the dorsal side periphery of the wearing article, the exterior can be kept simple and beauty can be maintained. By forming a through hole before cutting the absorbent body continuous body, the cutout part of the absorbent body can be formed with high precision, and a cutout part having a desired shape can be efficiently formed.

Preferably, in the absorbent body cutting process, a through hole is formed in the absorbent body continuous body so as to cross an intermediate portion of the boundary line of the second region of the absorbent body continuous body, and then, the absorbent body continuous body is cut along the boundary line, to form the absorbent body.

In this case, the through hole forms a cutout part in one end edge of the absorbent body. Compared to a case of forming a cutout part on one end edge of the absorbent body at the same time as cutting the absorbent body continuous body or a case of forming a through hole and cutting the absorbent body continuous body at the same time, it is easy to form a cutout part with high precision, and a cutout part having a desired shape can be efficiently formed.

### EFFECTS OF THE INVENTION

According to the present invention, a concave shape can be formed in a portion placed on the ventral side without forming or exposing a convex or concave shape on the periphery of a portion placed on the dorsal side, which can improve the wearing comfort and appearance.

### BRIEF DESCRIPTION OF THE DRAWING

[Figure 1] FIG. 1 is a schematic view of a wearing article. (Example 1)
[Figure 2] FIG. 2 is a schematic view of a manufacturing process of a wearing article. (Example 1)
[Figure 3] FIG. 3 is a schematic view of a manufacturing process of an absorbent body. (Example 1)
[Figure 4] FIG. 4 is a plan view of a wearing article. (Conventional Example 1)
[Figure 5] FIG. 5 is a plan view of a wearing article. (Conventional Example 2)

### MODE FOR CARRYING OUT THE INVENTION

Embodiments of the present invention will be described below with reference to the drawings.

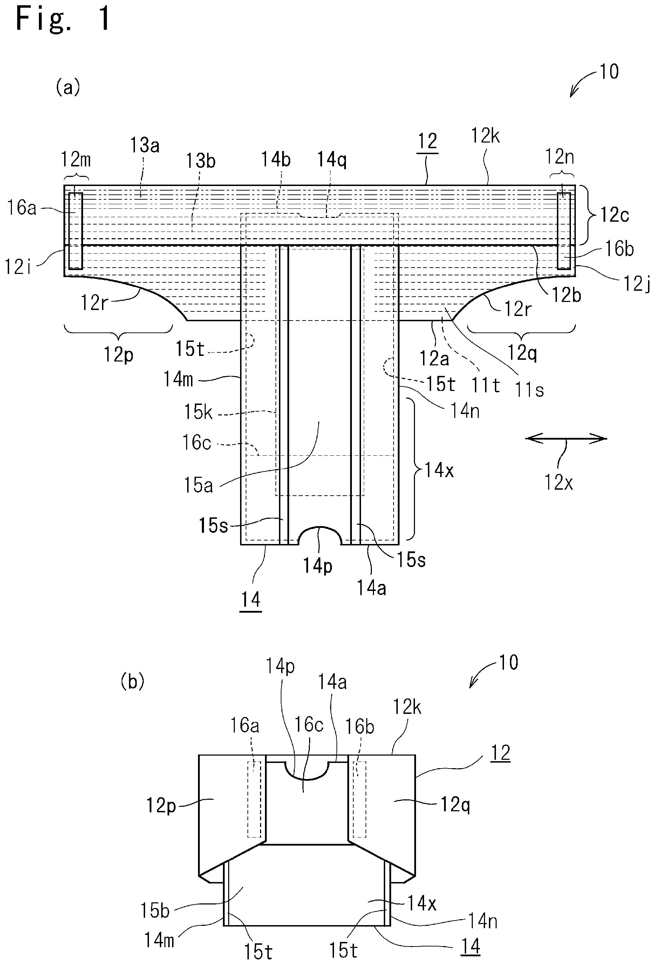

<Example 1> The wearing article 10 of Example 1 and its manufacturing method will be described with reference to FIGS. 1 to 3. FIG. 1(a) is a schematic view showing the developed state of the wearing article 10, and shows the skin side that comes into contact with the wearer's skin. FIG. 1(b) is a schematic view showing the wearing article 10 in a folded state.

As shown in FIG. 1, the wearing article 10 generally includes a girth member 12, an absorbent body 14, and fastening members 16a, 16b, and 16c.

The absorbent body 14 is formed into a substantially rectangular shape, and has a pair of end edges 14a, 14b facing each other, and a pair of side edges 14m, 14n facing each other. A cutout part 14p is formed in the intermediate portion of one end edge 14a. Further, another cutout part 14q is formed in the intermediate portion of the other end edge 14b. A configuration in which the other cutout part 14q is not formed may also be used. The depth of the cutout part 14p on one end edge 14a is preferably greater than the depth of the cutout part 14q on the other end edge 14b. The end edges 14a, 14b and the side edges 14m, 14n may be straight or curved.

The girth member 12 has a pair of side edges 12a and 12b extending in a first direction 12x indicated by an arrow 12x, and a portion 12c adjacent to the other side edge 12b is folded back. In this embodiment, cutout parts 12r are formed on both sides of one side edge 12a of the girth member 12, but the cutout parts 12r may not be formed.

In the developed state shown in FIG. 1(a), the absorbent body 14 is joined to the girth member 12 in a T-shape. That is, the absorbent body 14 is joined to the girth member 12 so that the other end edge 14b of the absorbent body 14 overlaps with the girth member 12, and the intermediate portion between one end edge 14a and the other end edge 14b of the absorbent body 14 overlaps with one side edge 12a of the girth members 12. A portion 12c of the girth member 12 adjacent to the other side edge 12b is folded back along the fold line 12k so as to cover the other end edge 14b of the absorbent body 14 and the cutout part 14q. The fold line 12k and the side edges 12a, 12b of the girth member 12 are parallel to each other. The folded portion 12c of the girth member 12 may be joined to another portion of the girth member 12, or may be joined to the absorbent body 14.

By folding-back the girth member 12, the other end edge 14b of the absorbent body 14 and its vicinity are covered by the folded portion 12c of the girth member 12, which can improve the wearing comfort and appearance. That is, since the other end edge 14b and the cutout part 14q of the absorbent body 14 do not touch the wearer's skin, discomfort can be reduced or eliminated. In addition, since the folded portion 12c of the girth member 12 touches the wearer's skin, the skin touch can be improved. Since the cutout part 14q of the other end edge 14b of the absorbent body 14 is not exposed, the exterior can be kept simple, beauty can be maintained, and the appearance can be improved.

The absorbent body 14 has a core 15k containing pulp or the like for absorbing liquid etc. disposed between a liquid-permeable sheet 15a placed on the skin side and a liquid-impermeable sheet 15b placed on the non-skin side. Three-dimensional gathers 15s are provided on the skin side of the absorbent body 14. The three-dimensional gathers 15s are configured, for example, to stand up when the absorbent body 14 is curved so that the side edges 14m and 14n of the absorbent body 14 are curved so that the skin side of the absorbent body 14 becomes concave. Further, leg gathers 15t are provided along side edges 14m and 14n of the absorbent body 14. A fastening member 16c is fixed to a portion adjacent to one end edge 14a of the absorbent body 14 so as to be exposed on the non-skin side.

The girth member 12 has stretchability in the first direction 12x. The girth member 12 is, for example, a stretchable composite member in which a plurality of two types of elastic stretchable members 13a, 13b with different characteristics are arranged between two nonwoven fabric sheets 11s, 11t so as to extend in the first direction 12x, and while the elastic stretchable members 13a, 13b are stretched, appropriate locations of the elastic stretchable members 13a, 13b and the nonwoven fabric sheets 11s, 11t are joined to each other. The elastic stretchable members 13a, 13b may be of one type, two or more types, or may be singular.

For example, thread rubber, a resin sheet having stretchability, or the like is used for the elastic stretchable members 13a and 13b. In the girth member 12, wrinkles are formed in the nonwoven fabric sheets 11s and 11t in a natural state in which the elastic stretchable members 13a and 13b are no longer stretched. When the girth member 12 is pulled in the first direction 12x, the elastic stretchable members 13a and 13b are stretched, and the wrinkles of the nonwoven fabric sheets 11s and 11t are stretched to smooth, and when the stretching is stopped, the member contracts.

Fastening members 16a and 16b are fixed to end parts 12m and 12n of the girth member 12 on both sides in the first direction 12x. The fastening members 16a, 16b may be fixed at an interval from the end edges 12i, 12j on both sides of the girth member 12 in the first direction 12x, or may be fixed so as to overlap with the end edges 12i, 12j. Furthermore, the fastening members 16a and 16b may be fixed so as to protrude from the girth member 12 beyond the end edges 12i and 12j, or may be fixed in a folded state.

For example, hook-and-loop fasteners are used for the fastening members 16a, 16b; 16c. The fastening members 16a and 16b fixed to the girth member 12 are first members 16a and 16b of hook-and-loop fasteners having hook surfaces raised in a hook shape, and are fixed so that the hook surfaces are exposed on the skin side. The fastening member 16c fixed to the absorbent body 14 is a second member 16c of a hook-and-loop fastener having a loop surface that is densely brushed in a loop shape, and is fixed so that the loop surface is exposed on the non-skin side. The hook surfaces of the fastening members 16a and 16b fixed to the girth member 12 and the loop surface of the fastening member 16c fixed to the absorbent body 14 can be removably adhered to each other, and adhesion and separation thereof can be repeated.

As shown in FIG. 1(b), the wearing article 10 can be folded and brought into a fastened state in which the girth member 12 is fastened to the absorbent body 14. That is, in the developed state shown in FIG. 1(a), the absorbent body 14 is folded back so that a portion 14x on one end edge 14a side of the absorbent body 14 overlaps with the other end edge 14b side of the absorbent body 14 and the folded-back portion 12c of the girth member 12. Next, as shown in FIG. 1(b), the flap portions 12p and 12q on both sides of the girth member 12 in the first direction 12x are folded back and overlapped on the non-skin surface side of the folded portion 14x of the absorbent body 14, and the fastening members 16a and 16b fixed to the girth member 12 are fastened to the fastening member 16c fixed to absorbent body 14. Thereby, the wearing article 10 is brought into a fastened state.

The fastening members 16a, 16b; 16c are members which make it possible that the end parts 12m, 12n on both sides of the girth member 12 in the first direction 12x are repeatedly fastened to and separated from the portion adjacent to one end edge 14a of the absorbent body 14. Although the fastening members 16a, 16b; 16c can be fixed to only one of the girth member 12 and the absorbent body 14, it is preferable to fix them to at least the girth member 12. It is also possible to use removable adhesive tape or the like for the fastening members 16a, 16b; 16c.

The wearing article 10 may be provided in the developed state shown in FIG. 1(a) or in the fastened state shown in FIG. 1(b).

In the latter case, the wearing article 10 is developed without releasing the fastening, to form a girth opening surrounded by the fold line 12k of the girth member 12 and two underbody openings surrounded by one side edge 12a and the cutout part 12r of the girth member 12 and the side edges 14m and 14n of the absorbent body 14, and both legs are passed from the girth opening to the underbody opening similarly to a pants-type diaper, thereby the wearing article 10 can be worn.

Moreover, since the fastening members 16a, 16b; 16c can be repeatedly fastened and separated, the wearing article 10 can also be worn similar to a tape-type diaper in the developed state as shown in FIG. 1(a) by releasing the fastening.

The wearing article 10 is worn with one end edge 14a of the absorbent body 14 placed in front of the wearer, and the girth member 12 wrapped around the wearer. At this condition, the cutout part 14p of one end edge 14a of the absorbent body 14 is placed at a position away from the wearer's navel, and the wearing article 10 can be aligned with respect to the navel so as not to touch the wound after cutting the umbilical cord.

Since the wearing article 10 can be unfastened and refastened after being worn, it is easy to accurately position the wearing article 10 with respect to the navel. Further, it is easy to adjust the girth fastening extent or the like, and to confirm whether or not the wearing article 10 needs to be replaced.

The wearing article 10 can form a concave shape in the portion disposed on the ventral side by the cutout part 14p of the one end edge 14a of the absorbent body 14. In the wearing article 10, since the fold line 12k of the girth member 12 becomes the periphery of the portion disposed on the dorsal side, no convex shape or concave shape is formed on the periphery of the portion disposed on the dorsal side. Therefore, the wearing article 10 can be easily packaged compactly.

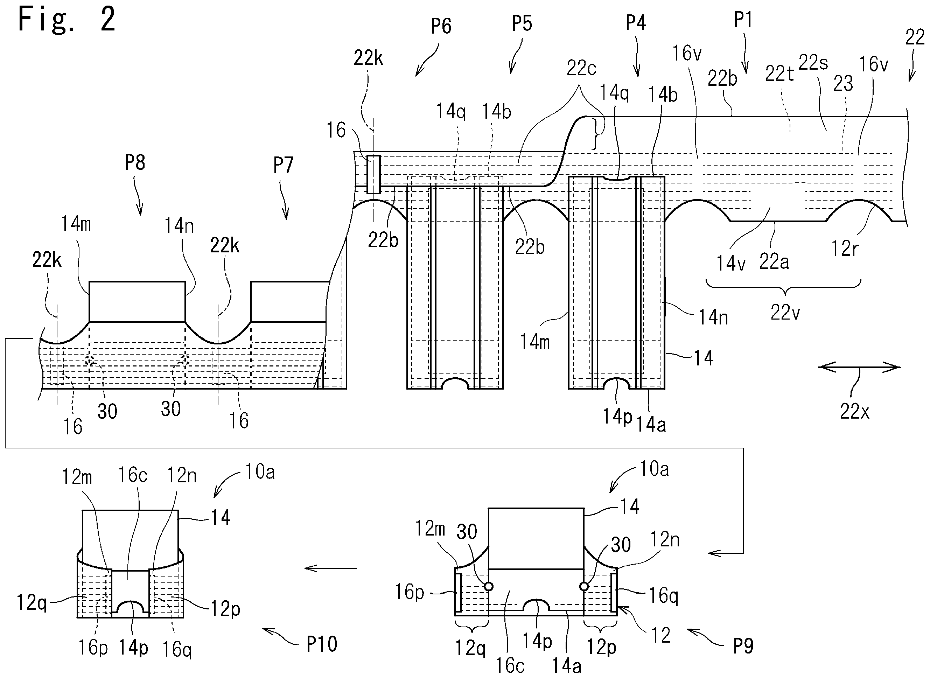

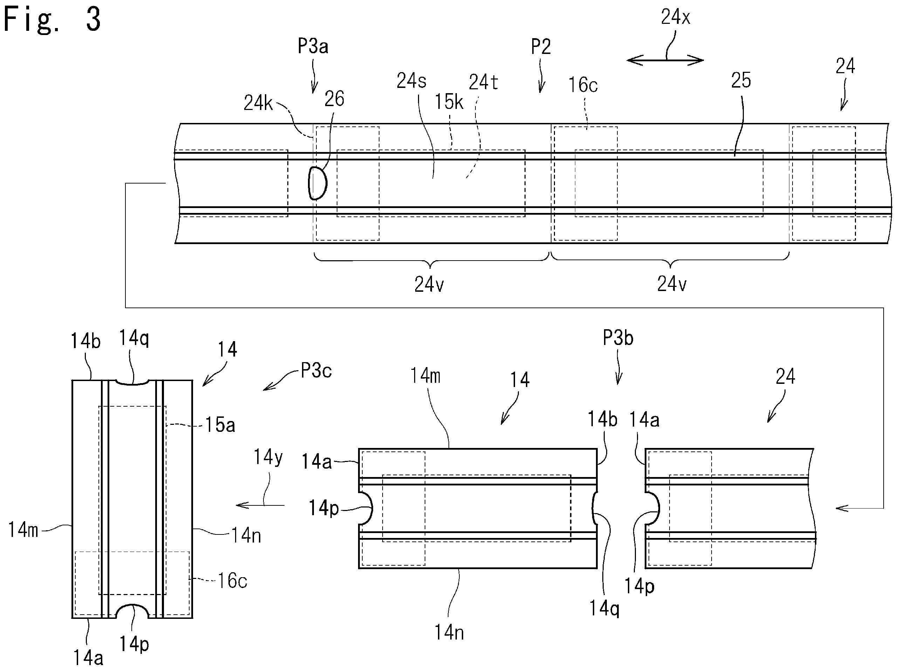

Next, a method of manufacturing the wearing article 10 will be described with reference to FIGS. 2 and 3. FIG. 2 is a schematic view of the manufacturing process of the wearing article 10. FIG. 3 is a schematic view of the manufacturing process of the absorbent body 14.
(i) First, a girth member continuous body 22 is prepared having a pair of side edges 22a, 22b extending in a first direction 22x indicated by an arrow 22x and in which a plurality of first regions 22v in which the girth member 12 is to be formed is connected in the first direction 22x, as indicated by the reference numeral P1 in FIG. 2. This is a girth member continuous body preparation process P1. The girth member continuous body 22 may be continuously formed and supplied to the subsequent process, or the girth member continuous body 22 formed and stored in advance may be supplied to the subsequent process.
   When continuously forming the girth member continuous body 22, for example, two nonwoven fabric sheets 22s and 22t wound into a roll are unwound, and the elastic stretchable member 23 in a stretched state is sandwiched between the nonwoven fabric sheets 22s and 22t, and in this state, the elastic stretchable member 23 and the nonwoven fabric sheets 22s and 22t are joined to each other at appropriate locations. Next, a cutter blade is inserted to cut the elastic stretchable member 23, so that tension of the elastic stretchable member 23 is generated in a portion indicated by a broken line 23, and tension of the elastic stretchable member 23 is not generated in regions 14v and 16v where the absorbent body 14 and the fastening member 16 are to be joined. Next, a cutout part 12r is formed on one side edge 22a. It is also possible to prepare the girth member continuous body 22 in which the cutout part 12r is not formed, and to form the cutout part 12r in a subsequent process.
(ii) In parallel with the girth member continuous body preparation process P1, an absorbent body continuous body 24 is prepared in which a plurality of second regions 24v in which the absorbent body 14 is to be formed are connected in a second direction 24x indicated by an arrow 24x, as shown by a reference numeral P2 in FIG. 3. This is an absorbent body continuous body preparation process P2. The absorbent body continuous body 24 may be continuously formed and supplied to the subsequent process, or the absorbent body continuous body 24 formed and stored in advance may be supplied to the subsequent process.
   When forming the absorbent body continuous body 24 continuously, for example, the continuous member 25 for forming three-dimensional gathers 15s (see, FIG. 1(a)) on the skin side is joined to a liquid-permeable sheet 24s which is being unwound from a roll and conveyed, to form a first continuous body, and additionally, a not-shown continuous member for forming leg gathers (see, FIG. 1(b)) and a fastening member 16c are joined to a liquid-impermeable sheet 24t which is being unwound from a roll and conveyed, to form a second continuous body. Next, while conveying the first continuous body and the second continuous body, cores 15k are sequentially arranged at a predetermined interval between the first continuous body and the second continuous body, and then, the first and second continuous bodies are joined to each other. It is also possible to prepare the absorbent body continuous body 24 to which the fastening member 16c is not fixed, and to fix the fastening member 16c to the absorbent body continuous body 24 or the absorbent body 14 in a subsequent process.
(iii) Next, the absorbent body continuous body 24 is cut to form an absorbent body 14 so that a pair of end edges 14a and 14b are formed on the absorbent body 14, and a cutout part 14p is formed in the intermediate portion of one end edge 14a, as shown by reference numerals P3a and P3b in FIG. 3. This is an absorbent body cutting process (P3a, P3b).

For example, in the absorbent body cutting process, a through hole 26 is formed in the absorbent body continuous body 24 so as to cross the intermediate portion of the boundary line 24k of the second region 24v of the absorbent body continuous body 24, as shown by a reference numeral P3a, and then the absorbent body continuous body 24 is cut linearly along the boundary line 24k, to form a substantially rectangular absorbent body 14 having end edges 14a, 14b and side edges 14m, 14n, as shown by a reference numeral P3b. Thereby, the cutout part 14p of one end edge 14a of the absorbent body 14 is formed by the through hole 26. The through hole 26 also forms a cutout part 14q on the other end edge 14b of the absorbent body 14.

Alternatively, in the absorbent body cutting process, the absorbent body continuous body 24 may be cut along a curved cutting line, so as to form a cutout part 14p on one end edge 14a of the absorbent body 14 simultaneously with the cutting of the absorbent body continuous body 24, although not shown. In this case, a convex portion corresponding to the contour shape of the cutout part 14p on one end edge 14a of the absorbent body 14 is formed on the other end edge 14b of the absorbent body 14, and this convex portion is covered by a portion 22c adjacent to the other side edge 22b of the girth member continuous body 22 by a folding back process P5 to be described later.

Alternatively, in the absorbent body cutting process, it is also possible to form the through holes 26 and cut the absorbent body continuous body 24 at the same time.

When forming a through hole 26 in the absorbent body continuous body 24 and then cutting the absorbent body continuous body 24 in a straight line as shown in FIG. 3, the stress distribution generated in the absorbent body continuous body 24 during processing becomes simple, as compared with a case of cutting the absorbent body continuous body 24 along the curved cutting line to form a cutout part 14p simultaneously with the cutting of the absorbent body continuous body 24, a case of effecting formation of a through hole 26 and cutting of the absorbent body continuous body 24 simultaneously, and the like. Therefore, it is easy to form the cutout part 14p with high accuracy, the absorbent body continuous body 24 can be conveyed at high speed to form the absorbent body 14, and the cutout part 14p having a desired shape can be efficiently formed.

When conveying the girth member continuous body 22 and the absorbent body continuous body 24 in parallel with each other, the absorbent body continuous body 24 is conveyed in the second direction 24x, and, as shown by a reference numeral P3c in FIG. 3, the direction of the cut absorbent body 14 is rotated by 90 degrees with respect to the conveying direction 14y of the absorbent body 14, and then supplied to the subsequent process.

(iv) Next, as shown by a reference numeral P4 in FIG. 2, a plurality of absorbent bodies 14 are joined to the girth member continuous body 22 at intervals with each other in the first direction 22x so that the other end edge 14b of the absorbent body 14 overlaps with the girth member continuous body 22, and the intermediate portion between the one end edge 14a and the other end edge 14b of the absorbent body 14 overlaps with one side edge 22a of the girth member continuous body 22. This is an absorbent body joining process P4. For example, the absorbent body 14 is joined to the girth member continuous body 22 by ultrasonic sealing or heat sealing. It is also possible to join the absorbent body 14 to the girth member continuous body 22 using an adhesive.

(v) Next, as shown by a reference numeral P5, a portion 22c adjacent to the other side edge 22b of the girth member continuous body 22 is folded back so as to cover the other end edge 14b of the absorbent body 14 joined to the girth member continuous body 22 and the other cutout part 14q. This is a folding back process P5.

(vi) Next, as shown by a reference numeral P6, the fastening member 16 is fixed to the girth member continuous body 22. This is a fastening member fixing process P6.

For example, a first member having a hook surface of a hook-and-loop fastener is used for the fastening member 16, and fixed to the girth member continuous body 22 in a state where it crosses the planned cutting position 22k of the girth member continuous body 22 indicated by a chain line 22k and the hook surface is exposed. The fastening member 16 is a member (16p 16q) that allows the end parts 12m and 12n of the girth member 12 to be repeatedly fastened to and separated from a portion adjacent to one end edge 14a of the absorbent body 14 (i.e., the fastening member 16c).

(vii) Next, as shown by a reference numeral P7, the girth member continuous body 22 and the other end edge 14b side of the absorbent body 14 are folded back, to fold the absorbent body 14 in half, and allowed to overlap with the one end edge 14a side of the absorbent body 14 so that the girth member continuous body 22 covers the one end edge 14a side of the body 14. The one end edge 14a side of the absorbent body 14 may be folded back, to fold the absorbent body 14 in half, and allowed to overlap with the other end edge 14b side of the absorbent body 14 so that the girth member continuous body 22 is covered by the one end edge 14a side of the body 14.

(viii) Next, as shown by a reference numeral P8, the parts near the side edges 14m and 14n on the one end edge 14a side of the folded absorbent body 14 and the girth member continuous body 22 are joined to each other to form a temporary fixing part 30. Since the absorbent body 14 is not opened from the half-folded state due to the temporary fixing portion 30, it becomes easy to transport the girth member continuous body 22 at high speed to which the half-folded absorbent body 14 is joined. Note that it is also possible to omit the formation of the temporary fixing portion 30.

(ix) Next, as shown by a reference numeral P9, the girth member continuous body 22, in which the portion 22c adjacent to the other side edge 22b is folded back, is cut at the planned cutting position 22k, to form a girth member 12 having end parts 12m and 12n on both sides of the first direction 22x. This is a girth member cutting process P9. At the same time as the girth member continuous body 22 is cut, the fastening member 16 is also cut, and the fastening member 16 is divided into two fastening members 16p and 16q. In the girth member cutting process P9, individual pieces of the wearing article 10a are formed.

(x) Next, as shown by a reference numeral P10, the flap portions 12p and 12q of the cut girth member 12 are folded back, and the divided fastening members 16p and 16q are fastened to the fastening member 16c fixed to the absorbent body 14. Thereby, the wearing article 10a in a fastened state in which both end parts 12m and 12n of the girth member 12 are fastened to the absorbent body 14 is completed.

If the processes indicated by reference numerals P7 to P10 are omitted and the girth member continuous body 22, in which the portion 22c adjacent to the other side edge 22b is folded back, is cut at the planned cutting position 22k, individual pieces of the wearing article 10a in a developed state can be formed, like the wearing article 10 shown in FIG. 1(a).

In the fastening member fixing process P6, after fixing the fastening members 16a and 16b on both sides of the planned cutting position 22k with an interval from the planned cutting position 22k, the girth member continuous body 22 is cut at the planned cutting position 22k, thereby a wearing article 10 can be formed in which the fastening members 16a and 16b are fixed apart from the end edges 12i and 12j of the girth member 12, as shown in FIG. 1(a).

The fastening member 16 can also be fixed to the girth member continuous body 22 before the portion 22c adjacent to the other side edge 22b of the girth member continuous body 22 is folded back. Moreover, it is also possible to fix the fastening members 16a and 16b to the girth member 12 after cutting. That is, in the fastening member fixing process, the fastening members 16; 16a, 16b are fixed to the girth member continuous body 22 or the girth member 12.

<Summary> As explained above, with the wearing articles 10, 10a, a concave cutout part 14p can be formed on the portion disposed on the ventral side without forming a convex or concave shape on the peripheral edge 12k of the portion disposed on the dorsal side, and wearing comfort and appearance can be improved.

Note that the present invention is not limited to the embodiments described above, and can be implemented with various modifications.

For example, the wearing article of the present invention can be manufactured in a variety of ways. For example, the wearing articles 10 and 10a can also be manufactured by cutting the girth member continuous body 22 to form the girth member 12 without folding-back the portion 22c adjacent to the other side edge 22b of the girth member continuous body 22, and by folding-back the portion 12c adjacent to the other side edge 12b of the girth member 12.

### DESCRIPTION OF REFERENCE NUMERALS

10,10a wearing article
12 girth member
12a one side edge
12b other side edge
12c adjacent part
12m, 12n end part
12x first direction
14 absorbent body
14a one end edge
14b other end edge
14p cutout part
14q other cutout part
16, 16a, 16b, 16c, 16p, 16q fastening member
22 girth member continuous body
22a one side edge
22b other side edge
22c adjacent part
22v first region
22x first direction
24 absorbent body continuous body
24k boundary line
24v second region
24x second direction
26 through hole
P1 girth member continuous body preparation process
P2 absorbent body continuous body preparation process
P3a, P3b absorbent body cutting process
P4 absorbent body joining process
P5 folding back process
P6 fastening member fixing process
P9 girth member cutting process

## Claims

1. A wearing article provided with
a girth member having a pair of side edges extending in a first direction,
an absorbent body having a pair of end edges facing each other, and in which a cutout part is formed in the intermediate portion of one end edge thereof, the absorbent body being joined to the girth member so that the other end edge overlaps with the girth member and the intermediate portion between the one end edge and the other end edge overlaps with one of the side edges of the girth member, and
fastening members enabling both end parts in the first direction of the girth member to repeatedly fasten to and separate from a portion of the absorbent body adjacent to the one end edge,
wherein a portion of the girth member adjacent to the other side edge is folded back so as to cover the other end edge of the absorbent body.

2. The wearing article according to claim 1, wherein
another cutout part is formed in an intermediate portion of the other end edge of the absorbent body, and
a portion of the girth member adjacent to the other side edge is folded back so as to cover the other cutout part.

3. A method of manufacturing a wearing article, comprising:
a girth member continuous body preparation process of preparing a girth member continuous body having a pair of side edges extending in a first direction, and in which a plurality of first regions in which girth members are to be formed are connected in the first direction;
an absorbent body continuous body preparation process of preparing an absorbent body continuous body in which a plurality of second regions in which absorbent bodies are to be formed are connected in a second direction;
an absorbent body cutting process of cutting the absorbent body continuous body to form the absorbent body so that a pair of end edges are formed on the absorbent body and a cutout part is formed on the intermediate portion of the one end edge;
an absorbent body joining process of joining a plurality of the absorbent bodies to the girth member continuous body at intervals from each other in the first direction so that the other end edge of the absorbent body overlaps with the girth member continuous body and the intermediate portion between the one end edge and the other end edge of the absorbent body overlaps with the one side edge of the girth member continuous body;
a folding back process of folding-back a portion of the girth member continuous body adjacent to the other side edge to cover the other end edge of the absorbent body joined to the girth member continuous body;
a girth member cutting process of cutting the girth member continuously body in which a portion adjacent to the other side edge is folded, to form the girth member having end parts on both sides of the first direction; and
a fastening member fixing process of fixing a fastening member enabling the end part of the girth member to repeatedly fasten to and separate from a portion of the absorbent body adjacent to the one end edge, to the girth member continuous body or the girth member.

4. The method of manufacturing a wearing article according to claim 3, wherein
in the absorbent body cutting process,
a through hole is formed in the absorbent body continuous body so as to cross an intermediate portion of the boundary line of the second region of the absorbent body continuous body, and then
the absorbent body continuous body is cut along the boundary line to form the absorbent body.
